# EUROPEAN PATENT APPLICATION

(11) **EP 4 740 920 A1**
(43) Date of publication of application: **13.05.2026**
(21) Application number: 24212445.1
(22) Date of filing: 12.11.2024
(51) Int. Cl.: A61F 13/505, A61F 13/15, A41B 9/12

(54) **RE-USABLE CHASSIS WITH REMOVAL TONGUE FOR USE WITH DISPOSABLE ABSORBENT PAD**

(71) Applicant: W. Pelz GmbH & Co. KG, 23812 Wahlstedt/Holstein (DE)
(72) Inventor: RÖTTGER, Henning, 24568 Kaltenkirchen (DE); WALLERT, Matthias, 23795 Klein Rönnau (DE)
(74) Representative: Plischke, Manfred

(57) **Abstract**

The present invention relates to a reusable hygiene article chassis comprising a pocket for receiving a replaceable absorbent pad. The chassis comprises a liquid intake zone which is particularly adapted to receive bodily exudates and further transfer these to the replaceable absorbent pad. The chassis further comprises a removal tongue, adapted to allow easy removal after use.

## Description

### Field of the Invention

The present invention relates to a reusable hygiene article chassis comprising a pocket for receiving a replaceable absorbent pad. The chassis comprises a liquid intake zone which is particularly adapted to receive bodily exudates and further transfer these to the absorbent pad. The chassis further comprises a removal tongue, adapted to allow easy easier insertion and removal after use.

### Background

Apart from washable cloth diapers, two-piece diaper systems comprising a reusable and washable outer layer and a disposable insert have long been used. Over the last decades, disposable diapers have significantly improved both ease of application and liquid handling performance, which further led to healthier skin of a wearer even at extended wear times, not only for babies but also for incontinent adults as well as for menstruating women. Especially for the latter two categories, absorbent pads are broadly used for lower liquid handling requirements, such as light to medium incontinence or lower menstrual flow conditions, whereby such pads are typically inserted into regular underwear, optionally attached thereto by releasable adhesive, or - mostly in hospitals or care facilities - elasticated pants. In particular for mobile people, discreetness during wear times and ease of replacement become highly relevant. However, with an increased environmental awareness, disposable products have come under scrutiny for using non-renewable resources and/or for an increase carbon footprint in the overall lifecycle, including treatment of the used products.

It is also known to design products in particular for menstrual products or very light incontinence products, being washable with a slightly increased absorbent capacity in the crotch region so as to accommodate small amounts of exudates. Such products may comprise a hydrophilic wad of fibers in the crotch region, optionally in combination with a thin protective coating or film to reduce the risk of soiling of the clothing. Such products may be laundered and reused many times, and as such also allow the use of more expensive materials, such as textile extensible materials, such as knitted cotton, as well known from conventional underwear, or elastic materials, such as rubber, preferably synthetic rubber, or synthetic elastic materials, known as LYCRA ^{™} and the like.

US6142983 discloses a disposable absorbent article with an outer cover, an absorbent chassis and an absorbent support member which is configured to maintain the absorbent chassis in close contact with the wearer's body in use. US20060247599 refers to an absorbent garment having a garment-like outer shell and an absorbent assembly positioned therein. WO1995010992A1 discloses a diaper assembly including a washable over-garment and a reusable moisture proof sheet positioned on the inner surface side of the garment.

US3489149 (Larson) describes a re-usable and washable panty for menstrual wear having in the crotch portion a pocket for an optional absorbent insert or pad, wherein the user-oriented portion of the pocket is formed from a first hydrophobic, yet moisture-passing layer for improved skin dryness oriented towards the wearer and a hydrophilic layer oriented towards the insert, if present, or the panty material, which has some tendency to retain moisture for the case when not used in combination with an insert pad.. The insert may be disposable.

US2021/0100698 (P&G, Langdon) describes a unitary or two-piece article, wherein the chassis portions exhibit particular stretch properties so as to allow good adaptation to body contours, especially in the crotch region.

In EP2376046, a wearable absorbent article to be worn by a wearer about the lower torso is described, comprising a re-useable chassis adapted to receive an absorbent insert therein. The chassis comprises insert fastener components and the absorbent insert comprises a pair of elasticized standing cuffs.

Along these developments, the commercially available PAMPERS ^{™} Hybrid design provides baby diapers with a reusable chassis and a disposable absorbent core - just as at the beginning of diapering, but with more modern materials, especially superabsorbent materials. WO2023/180584 Al (Pelz), hereinafter referred to as WO'584, describes a system with an article chassis that is adapted to receive an absorbent pad, so as to ease replacing of the pad upon loading, addressing improved positioning but also exchange of a loaded absorbent core

In WO2024/213393A1 (Pelz) an absorbent pad is described, that is suitable to be flushed, such as in a toilet and especially adapted to be fitted into a chassis, such that a hygiene article system is formed. In aiming at good disintegration after use, particular executions exhibit a low wet strength, e.g., in a preferred execution, the pad is essentially free of any adhesive or glue. However, lower wet strength may lead to more difficult removal of the pad after use.

Whilst such a system addressed the need for products that satisfy the desire for minimal disposable material together with good performance even at higher liquid loads and with customizable properties and functionalities that are incompatible with washing requirements, research identified room for improvement with regards to the ease of insertion and removal of the absorbent pad from the chassis.

### Summary

Henceforth, in a first aspect, the present invention is a re-usable article chassis for being worn on the lower torso of a wearer exhibiting a user-oriented inside surface and an opposite outer surface comprising
- at least one liquid intake zone adapted to cover a region of the exudate releasing body openings,
- a main chassis region preferably fully circumscribing the at least one liquid intake zone.

The article chassis further comprises at least one pad pocket covering at least a portion of the at least one intake zone and comprises a pad pocket opening, adapted for receiving and positioning an absorbent pad in liquid contact with the liquid intake zone.

The chassis further comprises a removal tongue connected, optionally releasably connected, with a fixed end to a front or rear portion of the chassis whilst the opposite end is essentially freely movable. The removal tongue is adapted to receive an absorbent pad upon donning and to allow pulling of the absorbent pad upon its removal.

The removal tongue may optionally comprise a material exhibiting at least one the elements selected from the group consisting of
a) - exhibiting
   a1) a liquid permeability;
   a2) an absorbency of less than about 4 g/g, or lea than about 2 g/g or less than about 1 g/g or less than about 1 g/g;
   a3) hydrophobicity;
b) - exhibiting a coefficient of static friction
   bi) of its surface oriented towards the pocket of less than 0.8 towards the chassis material;
   bii) of its surface opposite the one oriented towards the pocket of more than 0.2 towards the inserted absorbent core.

The tongue of the re-usable article chassis may comprise at least one material which is selected from the group consisting of
3a) a nonwoven material;
3b) an apertured film;
3c) a net;
3d) a textile fabric, preferably a woven or knitted fabric.

Preferably, the re-usable article chassis comprising the tongue is washable at at least 40°C, more preferably at at least 60°C, even more preferably at at least 90°C for at least 30 times, and most preferably at least 60 times.

A re-usable article chassis may comprise a pad pocket opening, which is open towards the inside of the chassis and wherein the removal tongue comprises a removal pocket at its opposite end. A re-usable article chassis may comprise a pad pocket opening, which is positioned on the opposite surface of the chassis, and the tongue is adapted to be positioned into the pad pocket of the article together with an absorbent pad.

In a second aspect, the present invention is a system for receiving bodily exudates, comprising
- a chassis according to any of the preceding claims,
- and an absorbent pad adapted to be inserted into the pad pocket of the chassis. Preferably, the absorbent pad of the system is flushable according to EDANA GD4.

Preferably, the absorbent pad of the system is flushable according to IWSFG standard.

The system may further comprise an insertion aid, as may optionally be flushable according to EDANA GD4 or IWSFG flushability standard, and which is adapted to ease insertion of the absorbent pad and the tongue into the pad pocket.

Optionally, the stiffening aid may be separate from the chassis and the absorbent pad, and adapted to be removed after insertion. Optionally, the stiffening aid is flushable according to EDANA GD4 or IWSFG and separate from the chassis and the absorbent pad, and adapted to be removed after insertion.

In yet another aspect, the present invention is a method for using a system for receiving bodily exudates comprising the steps of
a) - providing a chassis as described;
b) - providing an absorbent pad adapted to fit into the pad pocket of the chassis;
c) - positioning the loose end of the tongue outside of the chassis;
d) - positioning the absorbent pad into a cross-directional fold of the tongue, optionally a preformed fold, and inserting the absorbent pad together with the tongue into the pad pocket;
e) - removing the absorbent pad from the pad pocket of the chassis by pulling the tongue with the absorbent pad positioned in a fold of the tongue, out of the pad pocket;
f) - discarding the absorbent pad;
whereby the steps b) to f) are executed in the given order, and optionally repeatedly.

Optionally, step e) may be executed by pulling the loose end of the tongue to the outside of the chassis.

Optionally, step e) may be executed by
- positioning a finger between the chassis and the tongue;
- pulling the loose end of the longue from the proximity of the attached end by moving the finger from the chassis.

### Brief Description of the Figures

Fig. 1A to F depict a first execution of a chassis and a system according to the present invention.
Fig. 2A to E depict a second execution of a chassis and a system according to the present invention.
Fig. 3A to D depict further executional details of a chassis and a system according to the present invention.
Fig. 4A to B depict further executional options of a chassis according to the present invention.
Fig. 5A to C depict further options for execution the present invention.
Fig. 6 depicts a further element for a system according to the present invention.

The figures are schematically only, and not to scale. Same numerals refer to same or equivalent features or elements, single (`) or multiple (", "', ...) apostrophes indicate duplicate features, such a left and right or front and back, etc.

### Detailed description

Within the present context, absorbent hygiene articles are intended to be worn on the lower torso of a human wearer of any age, adapted to receive bodily exudates, especially liquid exudates, such as urine, menses, as are released in the vaginal region, or from the penis, towards the "intake zone" of the article positioned adjacent to the exudate releasing organ.

Generally, absorbent hygiene articles comprise an "absorbent core" for handling the bodily fluids, i.e., acquiring, distributing and storing these therein, and a "chassis" for holding the absorbent core in place on the body of a wearer. Absorbent articles may be donned and doffed by the user him- or herself, or by a care-taker, such as a parent for babies.

### Hygiene articles can be classified as

- "pants style" articles, which enclose the waist of the wearer firmly connected like a belt and may be executed circumferentially seamless, but which may include side seams, which further may comprise means for tearing these open for easy doffing. Typically, such articles are stretchable or at least extensible for good fit and for easing donning,
- or "taped articles" that comprise fastening means for connecting the front and rear parts around the waist at donning, though these may also be pre-closed during manufacturing, such
that they have a pants like appearance, but are easy to be opened and re-closed, if necessary. In order to adapt to the complex body contours, the chassis often exhibit "elasticity", such as induced by elastic elements like rubber, preferably synthetic rubber, strands, including LYCRA^{™} or similar, but also elastomeric polyolefins, as in films or non-woven. In addition, or alternatively, the chassis may comprise structurally "extensible" materials, such as well-known e.g., from knitted cotton underwear, wherein the yarns are essentially non-elastic, but the knitted structure can readily conform to the body contours, albeit at retractive forces that are typically lower than for elastic materials.

An "absorbent pad" generally consists of an absorbent core between an upper, user-oriented coversheet and a lower, oppositely positioned lower coversheet, and may be held in place on the body by other means, such as a specially adapted removable chassis, or regular underwear, optionally aided by non-permanent adhesives and/or "wings" as may be folded around the crotch portion of the underwear. Optionally, and for certain applications preferably, the absorbent pad may be flushable, i.e., it may be discarded via the toilet, if designed so as to not clog or otherwise disturb disposal and to be degradable in the respective sewage treatment system, preferably by being biodegradable according to flushability criteria, e.g., as developed by EDANA for "flushable wipes" and as may be adapted to apply to absorbent pads. A particularly suitable execution for flushable pads for being used together with a chassis is described in the above mentioned WO2024/213393A1, to which express reference is made as to the design of and suitable materials for a flushable pad.

The term "permanent" refers to a feature that remains essentially unchanged during the intended use, and as such may be a connection, that can only be disconnected destructively, or a treatment, such as a hydrophobization, that remains during the intended uses, although its effect may be reduced. A connection may be releasably permanent, such as typically known from mechanical fastening systems, also referred to as "hook-and-loop" systems, where small hook-like elements engage with filamentary structure, such as a knitted loop material, or a suitably open nonwoven A "single piece diaper" comprises an "absorbent core" firmly and permanently connected to a "chassis" by which the absorbent core is held in place on the body of the wearer.

For "two-piece systems", an absorbent pad comprising an absorbent core and the chassis are separate or at least can be readily separated, though "pre-combined systems", where the manufacturer combines the absorbent pad and chassis removably, may be used. The absorbent pad may be a "replaceable" absorbent pad as can be readily removed and for which a like component or a component providing similar or different functionality can be substituted. The chassis may be similar to the underwear, as well known e.g., from hospital "net-pants" holding any absorbent pad more or less accurately in place. The present invention is directed to a particular chassis for such a two-piece system, a system comprising such a chassis and an absorbent pad, and the use of an absorbent pad and a chassis.

The chassis of the absorbent hygiene articles comprises an "inner" or "wearer facing layer", forming the innermost surface which may be in direct contact with the skin during use, typically formed by a skin contact sheet or "topsheet", but may also include other elements such as liquid handling elements, in particular in the "intake zone" of the article corresponding to the fluid releasing portions of the body, or (single or multiple) leakage barrier cuffs, or inwardly positioned chassis layers in the waist or hip regions. The chassis also comprises an "opposite", outer, or "garment facing surface". Within the present context the terms "upper" / "inner" and "lower" / "outer" correspond to "towards the user" and "away from the user", respectively. The term "major surface" describes surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.

Within the present context, the absorbent pad may be "disposable", i.e., intended for a single use for then being discarded, optionally recycled, in an environmentally compatible and hygienic manner. In a particular execution, the absorbent pad may be flushable, i.e., adapted to be discarded into a toilet, where it may disintegrate and allows removal through the sewer system without clogging. Most preferably such flushable pads not only disintegrate rapidly, but ultimately degrade into carbon dioxide in the sewage water treatment units. As often such flushable pads exhibit relatively low strength and/or integrity when loaded, the present invention provides particular advantages.

Especially the chassis may be fully "re-usable", by being adapted to be washed many times, typically withstanding at least 30 , preferably at least 60 wash cycles without major deterioration of its properties and functionality, with conventional detergents and at at least 40°C, preferably at at least 60°C or more, and even more preferably at at least 90°C. Lower temperatures may be acceptable, if particular cleaning agents are employed, or if the materials of the articles have been particularly treated, such as by permanently incorporated biocides. "Limited re-usability" refers to articles that can be cleaned multiple times, though have a higher wear than the fully re-usable ones, and/or may be discarded like disposable articles after several, e.g., up to ten, use cycles. Examples for fully re-usable articles are so-called "menstruation pants" that exhibit a low to moderate absorbency as may be achieved by hydrophilic fibers, such as cotton fibers or yarns, at a higher basis weight in, optionally also around, the intake zone.

The above mentioned "net pants" for hospital use typically exhibit a limited re-usability, as they can withstand washing conditions, at least for a few times, but may also be discarded, e.g., if heavily soiled by faeces, and may also be torn open for ease of removal in such cases.

The principles of a two-piece systems are further explained in the above referenced WO2023/180584 (Pelz), abbreviated WO'584, to which express reference is made for the general design elements and the functionality of two-piece systems, to particular materials as may be suitable for the various elements of the chassis, to particular exemplary executions for an absorbent pad, and to various optional features, as long as a skilled person will not realize blatant design contradictions.

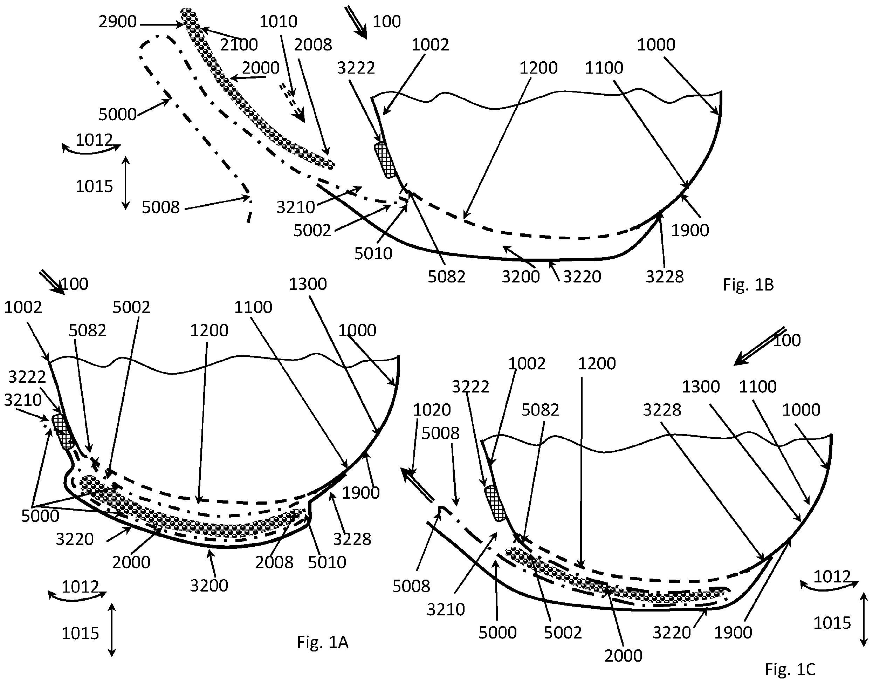

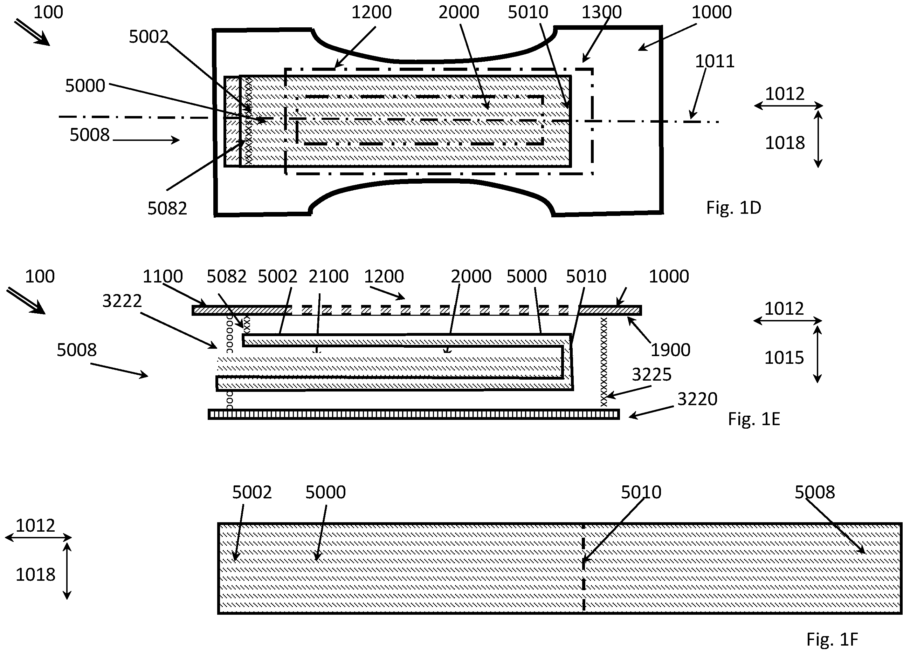

The particular elements of the present invention as well as their functioning are now explained by referring to Fig. 1 to 3, whereby the shown executions should not be seen limiting. Also, certain features or elements may be used independently from the other elements shown in the figures. In a first aspect, the present invention is a re-usable article chassis 1000, and in another one a two-piece hygiene article system, or kit, 100, comprising such an article chassis 1000 and an absorbent pad 2000, preferably as a single use absorbent pad, as exemplarily depicted in Fig. 1A, 2A, and 3A in an in-use configuration.

The article chassis 1000 exhibits a width (y-) 1018 and a length (x-) 1012 direction, the first corresponding to a left-right orientation on a user, the second corresponding to a line extending form the front, i.e., navel, through the crotch to the back, i.e., small of the back, of a user. A thickness (z-) direction 1015 extends perpendicularly to both. The chassis comprises a front (1002) and a rear (1008) end region, corresponding to the waist regions of a wearer.

The chassis 1000 is preferably executed in a "pants style", but may also be in an open diaper style. The chassis 1000 is a re-usable and washable article, and should exhibit superior fit properties, good softness, a nice hand and appearance, such as known from textile, e.g., knitted cotton, e.g., for underwear. It is highly preferred that the chassis, at least the main chassis region, is "breathable" (air permeable) by exhibiting air permeability values of more than about 0.2 m³/(m²*sec), or more than about 1.0 m³/(m²*sec), when tested according to ASTM D737.18. An essentially continuous wearer facing inner surface 1100 comprises a fluid intake zone 1200, which is adapted to allow passage of bodily exudates as deposited thereon to an absorbent pad 2000, and a main chassis region 1300, as may completely circumscribe the intake zone 1200. Whilst the shape of the intake zone 1200 is shown in the figures as a rectangle, it may also take other shapes, such as shown in more detail is WO'584, to which references is now made for the shapes and properties of the intake zone 1200.

The fluid intake zone 1200 may comprise a chassis fluid handling aid material that exhibits balanced liquid handling properties, i.e., good acquisition allowing released exudates to penetrate through and away from the wearer, as well as low rewet properties for a dry feel even after loading. Further, the chassis fluid handling aid material shall allow good liquid transfer of the loaded liquids to the absorbent pad and should preferably not exhibit a high absorbent capacity that would detrimentally affect washing and drying, as may be assessed by various property tests:
- The Single Load Drop Test with a penetration time of less than 30 sec, preferred 15 sec and most preferred less than 5 sec or even spontaneously penetrating the material.
- The "Nonwoven Run Off' values according to EDANA NWSP 080.9.R0 (15) are preferably less than about 50 %, or less than about 25 % or less than about 10 %, or even essentially 0 %.
- "Air Permeability of Nonwoven Materials" according to ASTM D 737-18 (measured at 125 Pa with a surface of 20 cm²) preferably more than about 0.5 m³/(m²*s), or more than about 1.0 m3/(m²*s) though typically less than about 3.0 m³/(m²*s).
- Aperturing, such as by apertures exhibiting a size corresponding to a circle having a diameter of more than about 0.25 mm, or of more than about 0.5 mm, or of more than about 1 mm or even more than about 5 mm, and typically less than about 15 mm or less than about 10 mm, whereby the apertures may also exhibit a three-dimensional funnel-like shape.
- "Repeated Liquid Strike-Through Time (Simulated Urine)", EDANA NWSP 070.7 (3 times 5 ml 0,9% NaCl) of less than 50 s, or less than 25 s, or less than 15 s or less than 10 sec.
- "Wetback After Repeated Strike-Through Time (Simulated Urine)" EDANA NWSP 070.8 of less than about 10.0 g, or less than about 5.0 g, or less than about 1 g or even less than about 0.5 g.

The chassis 1000 comprises a pad pocket 3200 positioned underneath the wearer facing surface 1100 and which is adapted to receive an absorbent pad 2000, whereby the application of the absorbent pad 2000 to the chassis 1000 may be made before donning of the combined chassis / absorbent pad system 100, but can also be readily performed after donning of the chassis without pad and/or when replacing a loaded pad. The chassis comprises a pocket opening 3210 for administering the absorbent pad, and a tongue 5000 for easier application and removal of the absorbent pad through the pad pocket opening 3210.

Fig 1 A to F refer to a first execution of the present invention, wherein Fig. 1A indicates an "in-use" configuration, Fig. 1B a "pre-use" configuration, and Fig. 1C, a "post use" configuration. Further, Fig. 1D depicts a top view of an in-use configuration, i.e., showing the length (1012) - width (1018) view of the overall contour of the chassis 1000 in a flat configuration, e.g., upon opening of the lateral (left / right) portions of the article, and Fig. 1E a cross-sectional view along the longitudinal center line 1011, with length (1012) and thickness (1015) dimensions, also of the in-use configuration stretched out flat. In Fig. 1D, the position of the fluid intake zone 1200 is indicated dashed-dotted line, and of the absorbent core 2000 thereunder in dashed-double-dotted outline.

In this execution, the pocket opening 3210 is towards the outside of the chassis, i.e., allowing access to the pocket 3200 from the outside, even when the article is donned. A pocket flap 3220, preferably liquid impervious, is connected with the chassis 1000 in the rear portion by a permanent rear pocket flap connection 3228, serving as a hinge to provide a pocket opening 3210 in the front portion, which may be releasably connected to the other chassis elements by front pocket flap connection 3222.

Further, the chassis 1000 comprises a tongue 5000, an elongate material that is permanently connected at its first, fixed end, here shown at its front end 5002 by a permanent or releasably permanent tongue connection 5082 to the outside 1900 of the chassis 1000 in the proximity of the front pocket opening 3210. The opposite end 5008 of the tongue 5000 is essentially unconnected and freely movable.

Referring to Fig. 1B, when applying a fresh absorbent pad 2000 with a user-oriented surface 2100 and an opposite surface 2900 to a chassis 1000 according to further aspect of the present invention, the tongue 5000 is pulled out of the pocket 3200 and over the opening 3210. The fresh absorbent pad 2000 is now pushed into the opening 3210, thereby engaging with the tongue 5000, thereby forming tongue fold 5010. Indicated by the dashed arrow 1010 along the insertion direction, the pad 2000 is now further pushed into the pocket 3200, and so is the tongue 5000 until the tongue fold 5010 is in the proximity of the rear pocket flap connection 3228. However, the length of the tongue 5000 is such that the loose end 5008 is still accessible from the outside, either by being positioned just inwardly of the pocket flap connection 3222, or, e.g., upon application of appropriate cut-out in the pocket flap and/or shaping of the tongue, outside of the pocket 3200. Thus, the side view in Fig. 1E depicts the tongue 5000 as an asymmetric laying U, covering the absorbent pad 2000 and with the end of the shorter (upper) leg of the U being connected to the chassis 1000 by front tongue connection 5082, and the opposite end 5008 may be peeking out of the opening 3210, or may stop just short of the pocket flap connection 3222, whilst Fig. 1F depicts top view of the full-length unfolded tongue 5000 with a fold line 5010.

Upon loading of the absorbent pad 2000, the front pocket flap connection 3222 may be opened and the loose end 5008 of the tongue can be pulled out, such that the absorbent pad 2000 can be readily extracted along the removal direction 1020 from the pocket 3200, see Fig. 1C. In particular for absorbent pads exhibiting a low wet strength, the pull forces are applied to the tongue rather than the pad, and the pad can be extracted without tearing or untimely disintegrating.

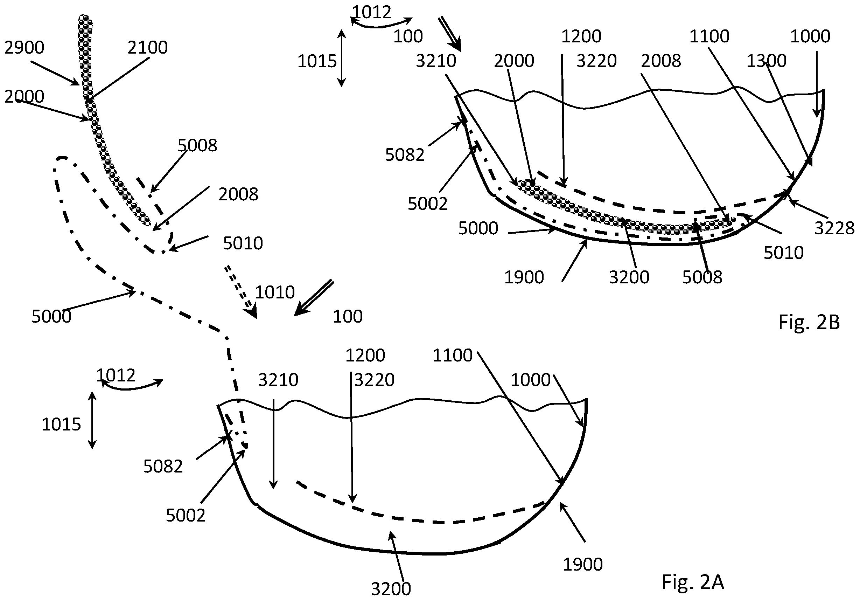

Referring to a second exemplary execution as shown in Fig. 2A to E, the absorbent pad 2000 may be inserted into the chassis 1000 through an inwardly positioned pad opening 3210, such that the pocket 3200 is formed on the user-oriented side 1100 of the chassis 1000 by a liquid permeable pocket flap 3220, with a permanent pocket flap connection 3228, here shown as a rearwardly positioned pocket flap connection, and preferably lateral permanent pocket flap connections 3225. As exemplarily shown, the pad pocket opening 3210 is a front pad pocket opening and for this execution, the elongate tongue 5000 is connected, preferably permanently, but optionally releasably permanent, with its front end 5002 towards the front portion of the chassis 1002. The opposite, loose end 5008 is formed into a removal pocket 5015, adapted to receive a rear portion 2008 of the absorbent pad 2000. It may be formed by overfolding the tongue material along a cross-directional fold line 5010, and connecting the lateral portions of the tongue with a lateral tongue connection 5085.

Upon donning, see Fig. 2A, the tongue 5000 is pulled out of the chassis 1000, and the fresh absorbent pad 2000 is inserted into the removal pocket 5015. Then, the absorbent pad 2000 in the removal pocket is inserted into the pad pocket 3200 of the chassis along insertion direction 1010. Upon appropriate selection of the tongue material, a tongue with a low coefficient of friction towards the inside surface of the outer chassis portion, the tongue may serve as an insertion aid, allowing easier slipping of the pad through the pad opening 3210 to arrive at the in-use-configuration, see Fig. 2B.

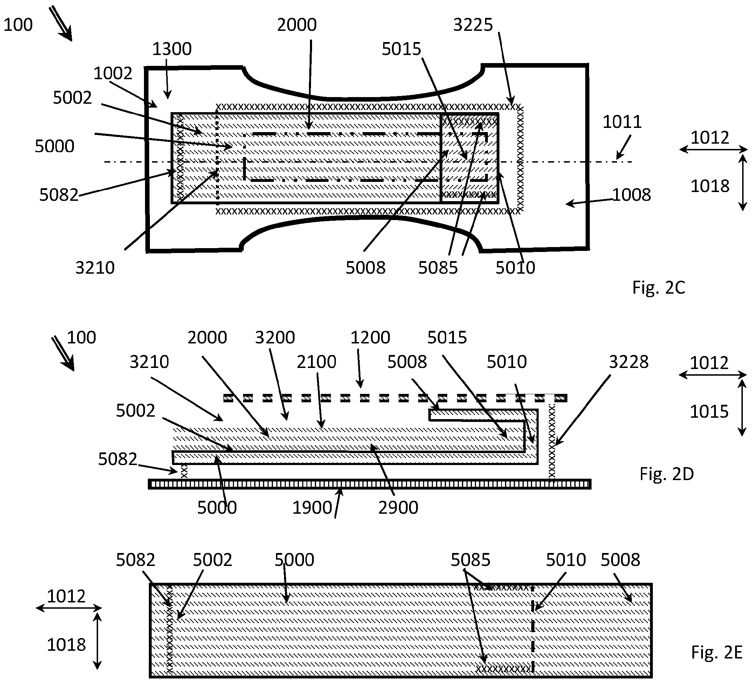

Fig. 2C depicts, in analogy to Fig. 1D, a top view of the chassis 1000 in the flattened in-use configuration, with the position of the intake zone 1200 depicted by the lateral (3225) and rearward (3228) pad pocket connections as "xxxx", and of the absorbent core 2000 in dash-double-dotted lines. Further, the tongue 5000 is shown with a front connection to the chassis 5082 and the rear removal pocket 5015 with lateral connections 5085.

In analogy to Fig. 1C, Fig. 2D depicts a side view along the longitudinal centerline 1011 of Fig. 2C, with the longer, lower leg of laying U of the tongue 5000 now being connected to the inside 1100 of the chassis, whilst the loose end 5008 of the tongue 5000 has the removal pocket 5015 with the absorbent pad 2000 being inserted in the in-use configuration. As indicated, the upper portion of the removal pocket 5015 may be in direct contact with pocket material 3220, such that the tongue may be made of material that may be impermeable or otherwise impede liquid transfer, as will be discussed in more detail herein below. Fig. 1E depicts the material of the tongue 5000 unfolded with the fold line 5010, and with the positioning of the lateral removal pocket connections 5085 and the front tongue connection 5082 indicated as "xx".

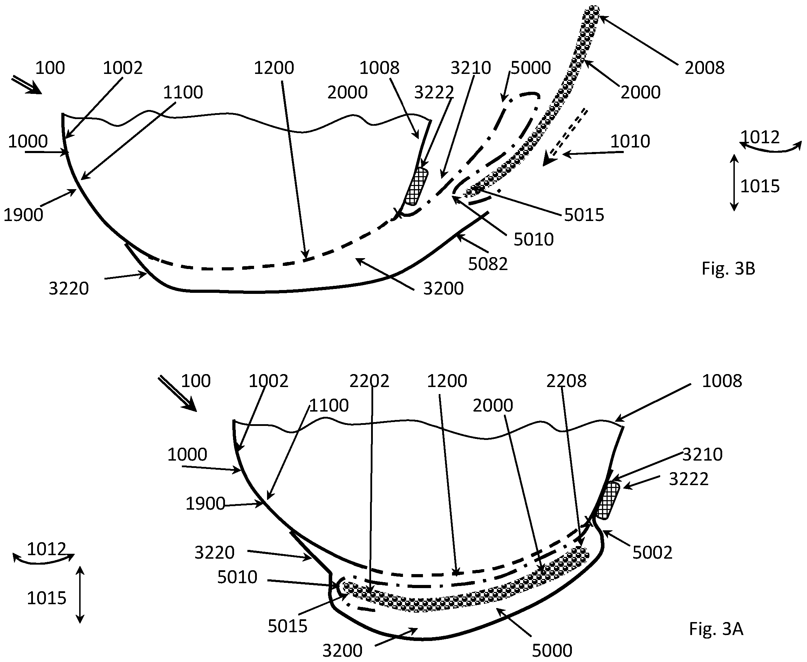

Yet a third exemplary execution is depicted in Fig 3 A to D, showing an system 100, whereby the pad pocket 3200 is accessible from the rear portion of the chassis, i.e., the pad pocket opening 3210 is towards the rear portion 1008 rather than towards the front portion 1002 as shown in Fig. 1 and 2. In this particular execution, an outer pad pocket 3200 is combined with a removal pocket 5015 of the tongue 5000, however - in contrast to the removal pocket as described in the context of Fig. 2 - the removal pocket is "downward", i.e., open towards the outer surface and the tongue 5000 is covering the full upper surface 2100 of the pad 2000 towards the intake zone 1200, and thus the tongue material should exhibit liquid handling properties as described in the context of Fig. 1, and further herein below.

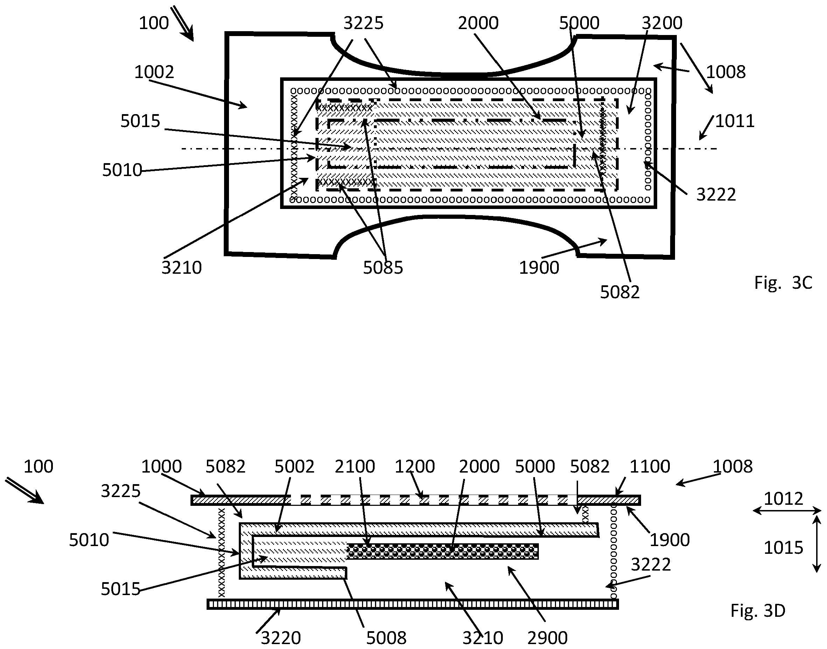

Thus, the three exemplified executions comprise the following design feature categories:
A - The pad pocket 3200 may be
   A1- an "outer pad pocket", as in the context of Fig. 1;
   A2 - or an "inner pad pocket", as in the context of Fig. 2;
B - The pad pocket opening 3210 may be
   B1 - towards the front 1002 of the chassis, as in the context of Fig. 1; 2;
   B2 - or towards the rear 1008 of the chassis, as in the context of Fig. 3;
   B3 - a single pocket with a front and a rear opening (not shown);
   B4 - a first pocket with a front opening and a second opening with a rear opening (not shown);
C - The tongue 5000 may be connected
   C1 - on the inner surface 1100 of the chassis 1000, as in the context of Fig. 2,
   C2 - or on the outer surface of the chassis, as in the context of Fig. 1, 3;
D - The tongue may
   D1- cover both surfaces (2100, 2900) of the pad 2000, as in the context of Fig 1;
   D2 - or comprise a removal pocket 5015, into which the crotch portion 2008 of the pad 2000 can be inserted and only one the surfaces of the pad is covered over the full length by the tongue 5000, whereby the covered surface is
      D2i - the upper, user-oriented surface 2100, as in the context of Fig. 3
      D2ii - or the opposite surface 2900 oriented away from the user, as in the context of Fig 2.

It is important to note that the present invention encompasses any combination of each one feature of the four categories A to D, for example a particular application may comprises the elements A2, B1, C2, and D2ii.

Having thus described the principles of the various aspects of the present invention, namely a particular design with a removal tongue for a chassis 1000, the system 100 of combining such a chassis 1000 with an absorbent pad 2000, and the method of inserting and extracting an absorbent pad 2000 from such a chassis 1000, the following describes further elements and features, as may be used alone or in combination with others.

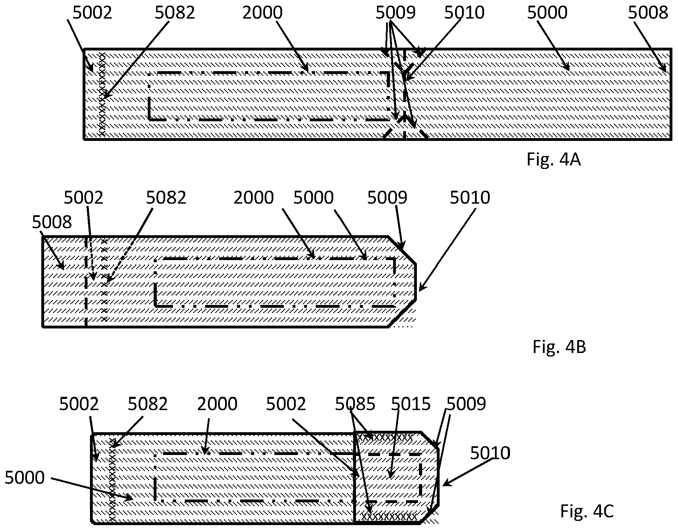

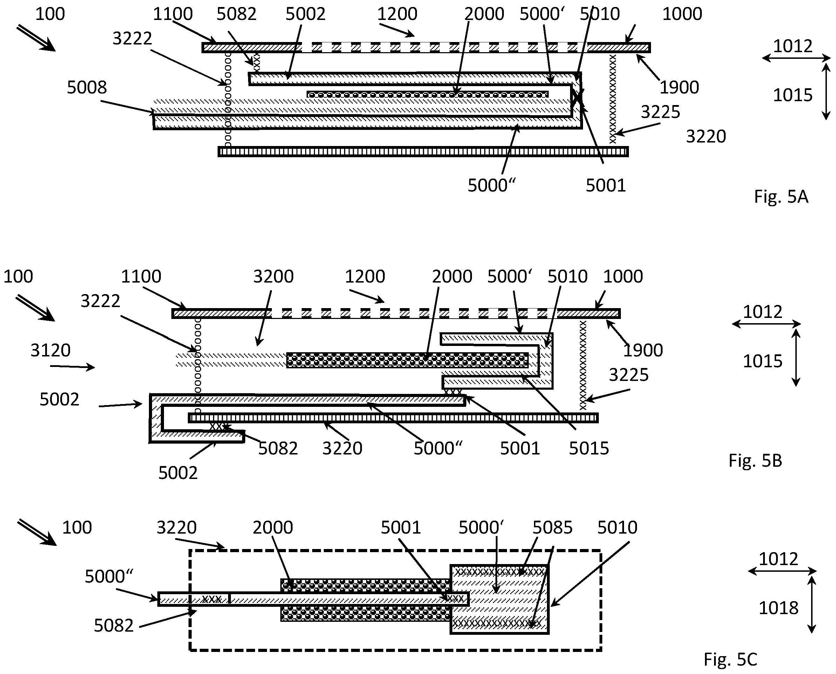

For simplicity of processing, the tongue may exhibit an elongate rectangular shape, as indicated in Fig. 1 through 3. However, different shapes may be contemplated, such as comprising rounded or chamfered corners, see Fig. 4A, B. The tongue 5000 may exhibit the same width as the core 2000, though a wider tongue, as indicated in Fig. 1 to 3, may ease insertion and removal, such as projecting cross-directionally more than about 2 mm, or more than about 5 mm, or even more than about 10 mm relative to the absorbent pad 2000. However, also a tongue 5000 with a width smaller than the absorbent pad may be employed, however, it should then be balanced with the wet strength or wet stiffness of the absorbent pad to avoid deformation of the pad upon removal. Whilst for simplicity of manufacturing the tongue may be made unitary from a single web, it may also be a composite material. As indicated in Fig. 5A in analogy to the design discussed in the context of Fig. 1E, the upper, user-oriented portion 5000' of the tongue may be a material particularly adapted to handle liquid, whilst the portion 5000" opposite thereto, relative to the absorbent pad 2000, may be a hydrophobic or liquid impermeable material, the two materials being connected to each other by a tongue seam 5001. The tongue seam may be executed by conventional means, such as glue bonding or thermal or ultrasonic bonding. Alternatively, the two portions may be of the same base material, which has been treated differently in different regions, e.g., hydrophilized or hydrophobized, respectively.

In Fig. 5B, as a cross-sectional view, and 5C as a view from the outer side, an option is shown, exhibiting two portions of tongue material, whereby the first tongue material 5000' forms just the removal pocket 5015 and is connected at a tongue connection 5001 to the second tongue portion 5000", which extends through the pocket opening 3210 towards the outer surface of the pocket material, connected thereto by tongue connection 5082. As indicated, the two portions may exhibit different widths.

The properties of the tongue material depend on the particular positioning of the tongue relative to the fluid intake zone and the absorbent pad.

In the executions, where tongue material is positioned between the fluid intake zone and the absorbent pad, at least this portion of the tongue material should not inhibit the fluid transfer to the absorbent pad, but may be adapted to improve liquid handling cooperatively with the material of the intake zone. Preferably, the fluid is quickly distributed to the absorbent pad, and the tongue material exhibits a low fluid absorbency to not give a wet feel, such as exhibiting less than about 50 g/m² or preferred less than 5 g/m²or most preferred less than 1 g/m², when tested according to EDANA NWSP 080.10.R0.

Optionally, the tongue material for any of the executions may be hydrophobic, or at least more hydrophobic than the main chassis region, to provide a dry feel when the user removes the pad by pulling the tongue, and may thus exhibit a vertical wicking height of less than 5 cm, or less than about 2 cm, or less than about 1 cm, or even essentially no vertical wicking at all. Optionally, the tongue material may exhibit balanced friction properties so as to allow easy insertion, such as by a low coefficient of friction at least of the surface contacting the backsheet or intake zone material. However, the friction should not be too low, as otherwise a slippery surface towards the core may result in displacement of the core. Thus, preferably, the coefficient of static friction of the tongue material towards the backsheet should be preferably less than 0.8 when tested according to ISO15359. The coefficient of static friction of the tongue material towards the absorbent core should be preferably more than 0.2 when tested according to ISO15359. In another execution, the tongue material may exhibit a higher friction towards the core, which may even be a directional friction, e.g., a low friction in the direction for inserting the pad, and a higher friction for the removal, as may be achieved by directional mechanical engagement hooks, as well known from mechanical fastener tapes.

For certain executions it may be preferred that the tongue material exhibits a balanced stiffness, so as to ease insertion without being uncomfortably stiff. Thus, the stiffness should be more than about 0.1 Nmm, or more than about 0,5 Nmm, or more than about 1.0 Nmm, or more than about 2 Nmm, and typically less than about 5.0 Nmm, when tested according to ASTM Standard Test Method D 1388, Option A, Cantilever test, or Option B, Heart Loop Test.

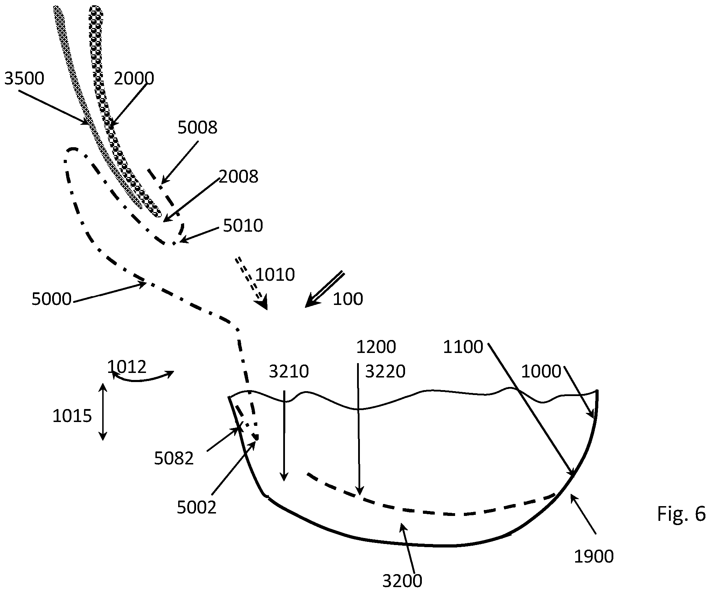

Optionally, an insertion aid may be provided to ease application of the absorbent pad, as described broadly in WO'584 to which express reference is made as far as the design of an insertion aid either integral with the absorbent pad, or separate, is made. Exemplarily, an insertion aid 3500 is shown in Fig. 6 in analogy to Fig. 2A with the additional insertion aid 3500, that may be placed into the removal pocket together with the absorbent pad 2000, but which may be removed once the pad 2000 is properly positioned for the in-use configuration. Such an insertion aid 3500 may be executed as a re-usable item, as it does not contact the soiled pad. However, it may also be made of material that loses its stiffness quickly upon wetting, such that it satisfies flushability requirements.

### Exemplary execution

A first example for a chassis article can be based on commercially available products, especially the Waschbare Inkontinenzhose MoliCare^{®} lady Absorbent underwear or Waschbare Inkontinenzhose MoliCare^{®} MEN Absorbent underwear, both of Paul Hartmann AG, Germany. A similar exemplary pants style product is made of
- a knitted chassis material of about 88% of polyester fibers and about 12% elastane at 165 g/m², or about 87% of cotton fibers and about 13% elastane at about 190 g/m².
- a topsheet of pure polyester fibers, of about 140 g/m²,
- a fluid barrier material of about 165 g/m²a polyamide fabric coated with a siloxane, e.g., polydimethylsiloxane,
- an absorbent fabric may comprise 80% polyester filaments and 20% polyamide filaments, at about 190 g/m².

Such articles comprised a pad pocket of about 250 mm length and 70 mm width for receiving a pad of about 50 mm width and 200 mm length.

Such chassis have been adapted by sewing a tongue material of an open polyester web material of about 50 g/m² at a width of 50 mm and a length of about 250 mm to the inside of the main chassis about 5 cm from the front waist end. The tongue formed a removal pocket of about 50 mm overfolded length at the lose end.

Such an article allowed easy application and removal of a disposable pad, even without contacting the absorbent pad.

### Test methods

All percentages are on a weight basis, unless expressly referred to otherwise.

All testing should be performed in a conditioned room maintained at about 23 +/- 2 °C and about 50 +/- 2 % relative humidity.

All equipment should exhibit an accuracy of at least one more digit than the reporting of the resulting measurements.

If a material exhibits a sidedness and the test results are susceptible to this, the test should be executed according to the intended "in-use" orientation.

"NWSP" refers to the "Nonwovens Standard Procedures", Edition 2015 (suffix R0.15), issued by EDANA, Belgium.
- "Repeated liquid strike through" - NWSP 070.7 & "wet-back after repeated strike through" (rewet) (NWSP 070.8) for nonwoven materials.
- "Water Vapor Transmission Rate" (WVTR) by the principle of measuring the time to increase humidity ("Lyssy") - Part 2, NWSP 070.6.
- "Vertical Wicking Test": A strip of sample material at a width of 50 mm is vertically hanged such that it is immersed 1 cm deep into a reservoir of 0.9% saline. The time is recorded after the sample is immersed and the corresponding weight change of the liquid in the reservoir is recorded. The reservoir is sufficiently large such that the change of fluid level is negligible. After 30 mins the Vertical Wicking Height has essentially reached a steady value, which is then recorded.
- "Air permeability": American Society for Testing and Materials (ASTM) D737-18 at 125 Pa and a surface of 20 cm².
- "Liquid Retention - Centrifuge Capacity": This test is generally following the EDANA NWSP 241.0.R2 (15) "Polyacrylate Superabsorbent Powders - Determination of the Fluid Retention Capacity in Saline Solution by Gravimetric Measurement Following Centrifugation," but applying it to an absorbent material, as per Test Method PA07/05 of Hygiene-Technologie GmbH (Hy-Tec), Haan, Germany. To this end, a test sample as treated according to the "Free Swell Absorption Capacity" test is positioned within a commercial centrifuge exerting a centrifugal force by having an internal diameter of 225 mm at a rotation of 2800 rpm) for at least 1 min. The test specimen is removed and weighed and the centrifuge absorbent capacity recorded in grams. For materials as being incorporated into products or articles, these are removed, such as by cutting, from the product or article.
- "Liquid Intake Time And Rewet"

The Acquisition / Rewet measurement of products designed for use in Adult Incontinence Test Method PA07/03 of Hygiene-Technologie GmbH (Hy-Tec), Haan, Germany.

The test employs an apertured Perspex plate of 70 mm by 220 mm with a central cylinder of an inner diameter of 20 mm and a height of 130 mm at a weight of 255 g, adapted such that weights of 2 kg each can be placed on onto the plate on either side of the cylinder. Upon placing the cylinder and the plate with weights centered on the plate, 50 ml of 0.9 w-% saline as synthetic urine are applied to the cylinder, and the acquisition time until complete disappearance of the liquid is recorded as 1^{st} gush acquisition time. 20 mins after that point in time, the second 50 ml are applied, and the 2^{nd} gush acquisition time until disappearance is recorded. After another 20 mins, the 3^{rd} 50 ml are applied and the 3^{rd} acquisition time is recorded. After 5 mins, the weights, plate and cylinder are removed and accurately (i.e., +/- 0.01 g or better) weighed 50 g of filter paper of 110 mm by 230 mm, are placed on the test specimen, followed directly by applying a plate without cylinder at 145 g and the 2*2 kg weights. After 15 secs, the weights and plate are removed and the rewetted filter paper is accurately weighed. The three acquisition times are added to the cumulative acquisition time in seconds, and the rewet value is recorded in grams.
- "Tensile properties": The tensile properties of the materials may be determined according to the NWSP 110.4, for a material width of 25 mm, length 150 mm, a gage length of 75 mm, and an extension speed of 300 mm / min.
- "Basis weight": NWSP 130.1, adapted to respective materials.
- "Standard Test Method for Stiffness of Fabrics" ASTM Standards D1388, Option A, "Cantilever Test", or Option B, "Heart Loop Test".
- "Nonwoven Thickness" The thickness for the samples was determined generally following EDANA Standard Procedure NWSP 120.1, with a circular pressure foot with 3 cm diameter at a pressure of 15g/m².

## Claims

1. A re-usable article chassis (1000) for being worn on the lower torso of a wearer exhibiting a user-oriented inside surface (1100) and an opposite outer surface (1900),
said article chassis (1000) comprising
- at least one liquid intake zone (1200) adapted to cover a region of the exudate releasing body openings,
- a main chassis region (1300) preferably fully circumscribing said at least one liquid intake zone; said article chassis (1000) further comprising at least one pad pocket (3200) covering at least a portion of said at least one intake zone (1200) and comprising a pad pocket opening (3210), adapted for receiving and positioning an absorbent pad (2000) in liquid contact with said liquid intake zone (1200),
wherein / **characterized in that**
said chassis (1000) further comprises a removal tongue (5000) connected, optionally releasably connected, with a fixed end (5002) to a front or rear portion (1002, 1008) of said chassis (1000) whilst said opposite end (5008) is essentially freely movable,
wherein said removal tongue (5000) is adapted to receive an absorbent pad (2000) upon donning and to allow pulling of said absorbent pad (2000) upon its removal.

2. A re-usable article chassis (1000) according to claim 1, wherein said removal tongue (5000) comprises a material exhibiting at least one the elements selected from the group consisting of
a) - exhibiting
a1) a liquid permeability;
a2) a liquid absorbency of less than about 50 ml/g, preferably less than about 5 ,l/g more preferably less than about 1 ml/g;
a3) hydrophobicity,
b) - exhibiting a coefficient of static friction
bi) of its surface oriented towards said pocket of less than 0.8 towards the chassis material;
bii) of its surface opposite the one oriented towards said pocket of more than 0.2 towards the inserted absorbent pad.

3. A re-usable article chassis (1000) according to claim 1 or 2, wherein
Said tongue comprises at least one material which is selected from the group consisting of
3a) a nonwoven material;
3b) an apertured film;
3c) a net;
3d) a textile fabric, preferably a woven or knitted fabric.

4. A re-usable article chassis (1000) according to any of the preceding claims, wherein said chassis (1000) comprising said tongue (5000) is washable at at least 40°C, preferably at at least 60°C, more preferably at at least 90°C for at least 30 times, preferably at least 60 times.

5. A re-usable article chassis (1000) according to any of the preceding claims, wherein said pad pocket (3200) of said chassis (1000) comprises an opening (3210), which is open towards the inside (1100) of the chassis (1000) and wherein
said removal tongue (5000) comprises a removal pocket (5015) at its opposite end (5008)

6. A re-usable article chassis (1000) according to any of the preceding claims, wherein said pad pocket (3200) of said chassis (1000) is positioned on said opposite surface (1900) of said chassis, and said tongue (5000) is adapted to be positioned into said pad pocket (3200) of said article together with an absorbent pad (2000).

7. A system (100) for receiving bodily exudates, said system (100) comprising
- a chassis (1000) according to any of the preceding claims,
- and an absorbent pad (2000) adapted to be inserted into said pad pocket (3200) of said chassis (1000).

8. A system (100) according to claim 7, wherein said absorbent pad is flushable according to EDANA GD4

9. A system (100) according to claim 7, wherein said absorbent pad is flushable according to IWSFG standard.

10. A system (100) according to any of claims 7 to 9, further comprising an insertion aid (3500), optionally being flushable according to EDANA GD4 or IWSFG flushability standard, adapted to ease insertion of said absorbent pad (2000) and said tongue (5000) into said pad pocket (3200).

11. A system (100) according to claim 10, wherein said stiffening aid is separate from said chassis and said absorbent pad, and adapted to be removed after insertion.

12. A system (100) according to claim 10 or 11, wherein said stiffening aid is flushable according to EDANA GD4 or IWSFG and separate from said chassis and said absorbent pad, and adapted to be removed after insertion.

13. A method for using a system (100) according to claim 7, said method comprising the steps of
a) - providing a chassis (1000) according to any of claims 1 to 6;
b) - providing an absorbent pad (2000), adapted to fit into said pad pocket (3200) of said chassis (1000);
c) - positioning the loose end (5008) of said tongue (5000) outside of said chassis (1000);
d) - positioning said absorbent pad (2000) into a cross-directional fold (5010) of said tongue (5000), optionally a preformed fold, and inserting said absorbent pad (2000) together with said tongue (5000) into said pad pocket (3200);
e) - removing said absorbent pad (2000) from said pad pocket (3200) of said chassis (1000) by pulling said tongue (5000) with said absorbent pad (2000) positioned in a fold (5010) of said tongue (5000), out of said pad pocket (3200);
f) - discarding said absorbent pad (2000);
whereby said steps b) to f) are executed in the given order, and optionally repeatedly.

14. A method for using a system (100) according to claim 13, wherein step e) is executed by pulling said loose end (5008) of said tongue (5000) to the outside of said chassis (1000).

15. A method for using a system (100) according to claim 13, wherein step e) is executed by
- positioning a finger between said chassis (1000) and said tongue (5000);
- pulling said loose end (5008) of said longue (5000) from the proximity of the attached end by moving said finger from said chassis (1000).
